# EUROPÄISCHE PATENTSCHRIFT

(11) **EP 4 074 720 B1**
(45) Veröffentlichungstag und Bekanntmachung des Hinweises auf die Patenterteilung: **19.07.2023**
(21) Anmeldenummer: 21168814.8
(22) Anmeldetag: 16.04.2021
(51) Int. Cl.: C07F 9/6574, B01J 31/00, C07C 45/50

(54) **GEMISCH VON BISPHOSPHITEN MIT EINEM OFFENEN UND EINEM GESCHLOSSENEN FLÜGELBAUSTEIN UND DESSEN VERWENDUNG ALS KATALYSATORGEMISCH IN DER HYDROFORMYLIERUNG**
MIXTURE OF BISPHOSPHITES WITH AN OPEN AND A CLOSED VOLATILE BUILDING BLOCK AND ITS USE AS CATALYST MIXTURE IN HYDROFORMYLATION
MÉLANGE DE BISPHOSPHITES DOTÉS DES ÉLÉMENTS STRUCTURAUX OUVERT ET FERMÉ ET SON UTILISATION EN TANT QUE MÉLANGE CATALYSEUR DANS L'HYDROFORMYLATION

(43) Veröffentlichungstag der Anmeldung: 19.10.2022
(73) Patentinhaber: Evonik Operations GmbH, 45128 Essen (DE)
(72) Erfinder: SALE, Anna Chiara, 45657 Recklinghausen (DE); FRANKE, Robert, 45772 Marl (DE); BRÄCHER, Alexander, 45721 Haltern am See (DE); FRIDAG, Dirk, 45721 Haltern am See (DE); KNOSSALLA, Johannes, 46514 Gahlen (DE); KUCMIERCZYK, Peter, 44628 Herne (DE); MARKOVIC, Ana, 45721 Haltern am See (DE)
(74) Vertreter: Evonik Patent Association

(56) Entgegenhaltungen:
- EP-A2- 0 213 639
- WO-A1-2008/115740
- JP-A- H08 165 266
- ANNEMIEK VAN ROOY ET AL: "Bulky Diphosphite-Modified Rhodium Catalysts: Hydroformylation and Characterization", ORGANOMETALLICS, Bd. 15, Nr. 2, 1. Januar 1996 (1996-01-01) , Seiten 835-847, XP055655992, ISSN: 0276-7333, DOI: 10.1021/om950549k

## Beschreibung

Die Erfindung betrifft ein Gemisch von Bisphosphiten mit einem offenen und einem geschlossenen Flügelbaustein und dessen Verwendung als Katalysatorgemisch in der Hydroformylierung.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigem Phosphor P^{III}. Eine gute Übersicht über den Stand der Hydroformylierung von Olefinen findet sich in R. Franke, D. Selent, A. Börner, "Applied Hydroformylation", Chem. Rev., 2012, DOI:10.1021/cr3001803.

In EP 0213639 A2 werden Bisphosphitliganden und ihre Verwendung in Übergangsmetallkomplex-katalysierten Reaktionen beschrieben. Die Bisphosphitliganden können hierbei eine Diorganophosphitfunktionalität oder eine Triorganophosphitfunktionalität aufweisen.

In WO 2008/115740 A1 wird ein kontinuierliches Hydroformylierungsverfahren zur Herstellung einer Mischung von Aldehyden beschrieben. Das Verfahren beinhaltet die Umsetzung einer oder mehrerer olefinisch ungesättigter Verbindungen mit Kohlenmonoxid und Wasserstoff in Gegenwart eines Organopolyphosphitliganden und eines Organomonophosphitliganden, wobei mindestens einer dieser Liganden an ein Übergangsmetall gebunden ist.

In JP H08 165266 A werden unterschiedliche Organophosphitliganden beschrieben, welche als Liganden in der Katalyse eingesetzt werden.

Die technische Aufgabe der Erfindung ist die Bereitstellung eines Ligandengemisches, welches in der Hydroformylierung von Olefinen eine gute n/iso-Selektivität aufweist und auch eine gute Ausbeute liefert.

Die Aufgabe wird gelöst durch ein Gemisch gemäß Anspruch 1.

Gemisch umfassend die Verbindungen (**1A**) und (**1B**):

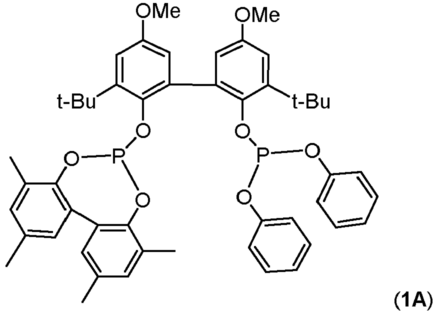

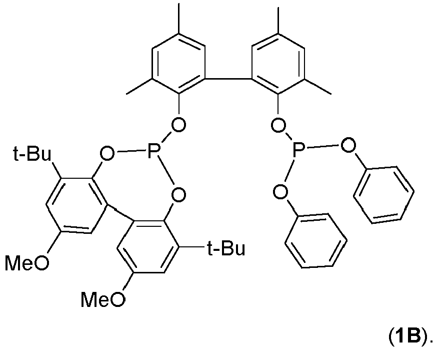

In einer Ausführungsform liegt der Gehalt an Verbindung (**1A**) in einem Bereich von 99,5 bis 0,5 Massen-%, der Gehalt an Verbindung (**1B**) in einem Bereich von 0,5 bis 99,5 Massen-%.

In einer Ausführungsform liegt der Gehalt an Verbindung (**1A**) in einem Bereich von 75 bis 25 Massen-%, der Gehalt an Verbindung (**1B**) in einem Bereich von 25 bis 75 Massen-%.

In einer Ausführungsform ist der Gehalt an Verbindung (**1A**) bezogen auf Massen-% größer, als der Gehalt an Verbindung (**1B**).

Neben dem Gemisch als solches wird auch dessen Verwendung zur Katalyse einer Hydroformylierungsreaktion beansprucht.

Verwendung eines zuvor beschriebenen Gemisches zur Katalyse einer Hydroformylierungsreaktion.

Des Weiteren wird ein Verfahren beansprucht, in welchem ein zuvor beschriebenes Gemisch als Ligandengemisch eingesetzt wird.

Verfahren umfassend die Verfahrensschritte:
a) Vorlegen eines Olefins,
b) Zugabe eines zuvor beschriebenen Gemisches und einer Substanz, welche ein Metall ausgewählt aus: Rh, Ru, Co, Ir aufweist,
c) Zuführen von H₂ und CO,
d) Erwärmen des Reaktionsgemisches aus a) bis c), wobei das Olefin zu einem Aldehyd umgesetzt wird.

In einer bevorzugten Ausführungsform ist das Metall Rh.

In einer Variante des Verfahrens ist die Substanz in Verfahrensschritt b) ausgewählt aus: Rh(acac)(CO)₂, [(acac)Rh(COD)] (Umicore, acac = Acetylacetonat-Anion; COD = 1,5-Cyclooctadien), Rh₄CO₁₂.

Es kann hierbei auch ein Überschuss an Liganden verwendet werden und nicht zwangsläufig jeder Ligand liegt gebunden in Form eines Ligand-Metall-Komplexes vor, sondern ist als freier Ligand im Reaktionsgemisch enthalten.

Die Reaktion wird bei üblichen Bedingungen durchgeführt.

Bevorzugt sind eine Temperatur von 80 °C bis 160 °C und ein Druck von 10 bis 60 bar.

Besonders bevorzugt sind eine Temperatur von 100 °C bis 140 °C und ein Druck von 20 bis 50 bar.

Die Edukte für die Hydroformylierung gemäß dem Verfahren der Erfindung sind Olefine oder Gemische von Olefinen, insbesondere Monoolefine mit 2 bis 24, bevorzugt 3 bis 16, besonders bevorzugt 3 bis 12 Kohlenstoffatomen mit end- oder innenständigen C-C-Doppelbindungen, wie z.B. 1-Propen, 1-Buten, 2-Buten, 1- oder 2-Penten, 2-Methyl-1-buten, 2-Methyl-2-buten, 3-Methyl-1-buten, 1-, 2- oder 3,-Hexen, das bei der Dimerisierung von Propen anfallende Cs-Olefingemisch (Dipropen), Heptene, 2- oder 3-Methyl-1-hexene, Octene, 2-Methylheptene, 3-Methylheptene, 5-Methyl-2-hepten, 6-Methyl-2-hepten, 2-Ethyl-1-hexen, das bei der Dimerisierung von Butenen anfallende Cs-Olefingemisch (Di-n-buten, Di-iso-buten), Nonene, 2- oder 3-Methyloctene, das bei der Trimerisierung von Propen anfallende C₉-Olefingemisch (Tripropen), Decene, 2-Ethyl-1-octen, Dodecene, das bei derTetramerisierung von Propen oder der Trimerisierung von Butenen anfallende C₁₂-Olefingemisch (Tetrapropen oder Tributen), Tetradecene, Hexadecene, das bei der Tetramerisierung von Butenen anfallende C₁₆-Olefingemisch (Tetrabuten) sowie durch Cooligomerisierung von Olefinen mit unterschiedlicher Anzahl von Kohlenstoffatomen (bevorzugt 2 bis 4) hergestellte Olefingemische.

Mit dem erfindungsgemäßen Verfahren können unter Verwendung der erfindungsgemäßen Liganden α-Olefine, endständig verzweigte, innenständige und innenständig verzweigte Olefine hydroformyliert werden.

Im Folgenden soll die Erfindung anhand eines Ausführungsbeispiels näher erläutert werden.

### Arbeitsvorschriften

### Allgemeine Analytik

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet.

Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR³¹P = SR¹H * (BF³¹P / BF¹H) = SR¹H * 0,4048.

### Synthese Vorschriften

### Vorstufen (3A) und (3B):

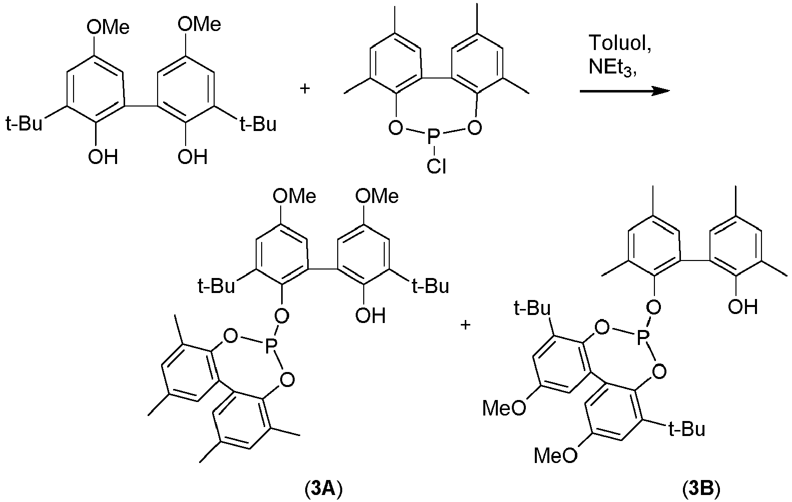

In einem 500 ml Schlenk wurden 10,7 g (0,03 mol) von 3,3'-Ditert.-butyl-2,2'-dihydroxy-5,5'-dimethoxy-biphenyl vorgelegt und mit 125 mL Toluol unter Rühren versetzt. Es entstand eine Suspension. In der Glovebox wurden 10 g von dem Chlorophosphit in einem 250 mL Schlenk eingewogen und ausgeschleust. Das Chlorophosphit wurde ebenfalls unter Rühren in 125 mL Toluol gelöst und mit (0,035 mol) EtsN versetzt. Die hergestellte Chlorophosphit-Base-Toluol-Lösung wurde anschließend unter kräftigem Rühren innerhalb von 1 h bei Raumtemperatur zur 3,3'-Ditert.-butyl-2,2'-dihydroxy-5,5'-dimethoxy-biphenyl Suspension tropfenweise zugegeben. Danach wurde der Reaktionsmixtur für 24 h gerührt. Anschließend wurde das Hydrochlorid abgefrittet und das Filtrat mittels Ölpumpenvakuum bis zur Trockne eingeengt. Dann wurde der Feststoff in die Glove-Box eingeschleust.

Gesamte Ausbeute 92%. Hiervon Isomer A: 64,3 Massen-%, Isomer B. 35,7 Massen-%.

### Vorstufen (4A) und (4B):

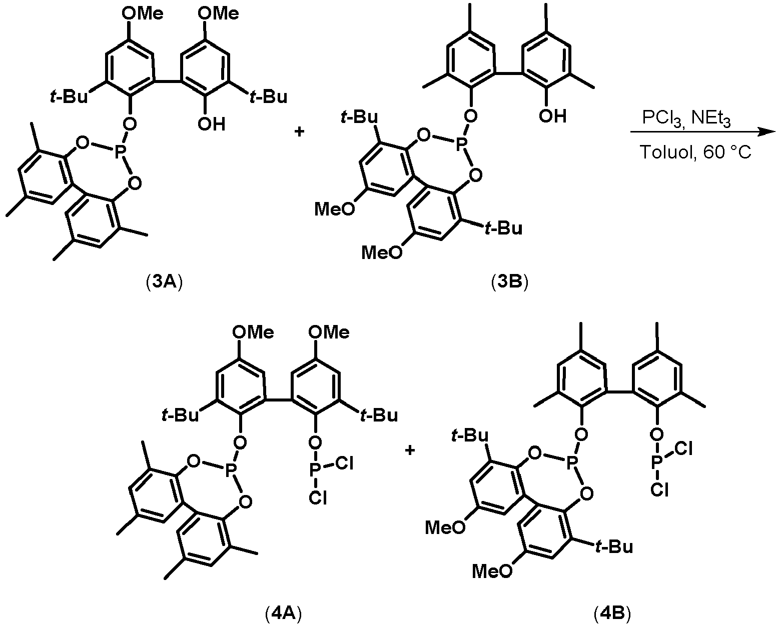

In einem sekurierten 250 mL Schlenk wurde in der Glove-Box 19 g (0,027 mol) des zuvor erhaltenen Isomerengemisches aus (**3A**) und (**3B**) abgewogen. Nach der Herausnahme aus der Glove-Box wurde zum Feststoff unter Rühren 160 mL getrocknetes Toluol und mittels Argon gespülter Spritze 12 mL = 8,8 g (0,086 mol) entgastes Triethylamin hinzugegeben. In einem zweiten 500 ml Schlenk wurde zunächst 100 mL getrocknetes Toluol vorgelegt und dann mittels Argon gespülter Spritze unter rühren 7,7 mL = 12 g (0,086 mol) Phosphortrichlorid zugegeben. Nun wurde unter kräftigem Rühren tropfenweise zu der Phosphortrichlorid/Toluol-Lösung die zuvor hergestellte Phosphit/Amin/Toluol-Lösung innerhalb von 25 Minuten bei Raumtemperatur zugeben. Nach vollständiger Zugabe wurde die Reaktionsmixtur auf 60 °C erwärmt und über Nacht bei dieser Temperatur gerührt. Nach Abkühlung auf Raumtemperatur wurde das entstandene Aminhydrochlorid abgefrittet. Das Filtrat wurde mittels Ölpumpenvakuum bei 55 °C bis zur Trockne eingeengt, der erhaltene Feststoff über Nacht nachgetrocknet.

Gesamte Ausbeute 90%. Hiervon Isomer A: 64,7 Massen-%, Isomer B. 35,3 Massen-%.

### Gemisch aus (1A) und (1B):

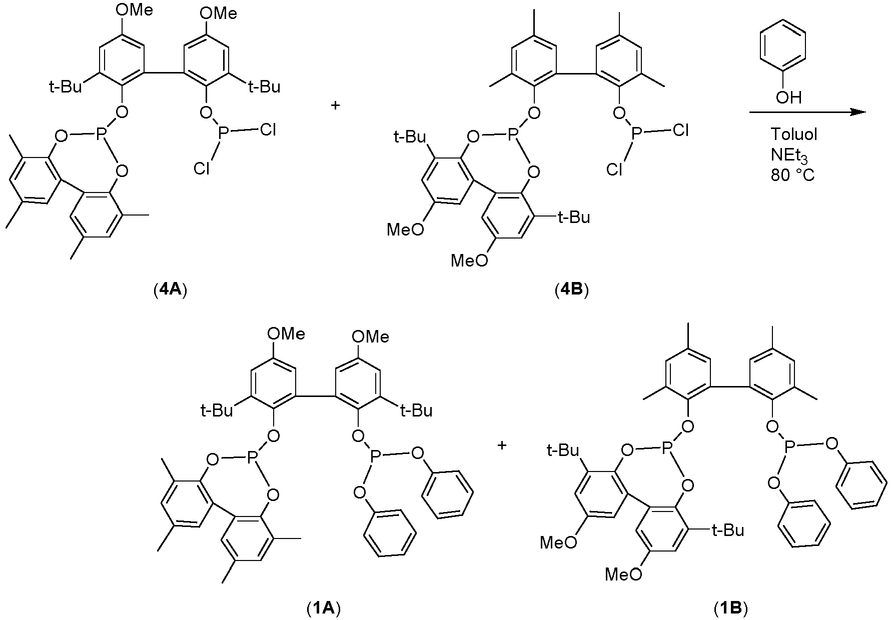

In der Glove-Box wurde in einem sekurierten 250 ml Schlenk 8 g (0,01 mol) Diorganophosphitdichlorphosphit-Gemisch aus (**4A**) und (**4B**), wie es zuvor erhalten wurde, abgewogen, anschließend ausgeschleusst und in 75 mL getrocknetem Toluol gelöst. In einem zweiten sekurierten 250 ml Schlenk wurde 1,9 g (0,02 mol) Phenol abgewogen und über Nacht mittels Ölpumpenvakuum bei Raumtemperatur nachgetrocknet. Am Morgen wurde unter rühren 50 ml getrocknetes Toluol und 6 mL = 4,4 g (0,044 mol) entgastes Triethylamin zugegeben und unter Rühren aufgelöst. Zur Phenol-Triethylamin Lösung wurde dann die zuvor hergestellte Chlorophosphit-Lösung in einem Zug hinzugegeben. Im Anschluss wurde die Reaktionsmixtur sofort auf 80 °C erwärmt und über Nacht bei dieser Reaktionstemperatur gerührt. Nach dem Abkühlen auf Raumtemperatur wurde das entstandene Aminhydrochlorid bei Raumtemperatur abgefrittet. Zur besseren Filtrierbarkeit des Aminhydrochlorid wurde zuvor der Rührer abgestellt und die Reaktionsmischung 1,5 h stehen gelassen. Das erhaltene Filtrat wurde bis zur Trockne eingeengt und über Wochenende mittels Ölpumpenvakuum bei Raumtemperatur nachgetrocknet. Der erhaltene Feststoff wurde unter rühren mit 100 ml entgastem Heptan versetzt und auf 70 °C erwärmt. Die Mischung wurde bei 70 °C, 1,5 h gerührt und unter rühren auf Raumtemperatur gebracht. Die getrübte Lösung wurde gefrittet. Das klare Filtrat bis zur Trockne eingeengt.

### Aufreinigung:

Um eine Chlorreduzierung zu erzielen, wurde das Produkt in ca. 20 ml getrocknetem Toluol gelöst. Anschließend wurde die Substanz über eine Fritte, die mit Silikagel gefüllt war unter Argon Atmosphäre filtriert. Das Silikagel wurde vorher in einem 600 ml Becherglas mittels getrocknetem Toluol aufgeschlemmt und in die Fritte überführt. Als Laufmittel wurde getrocknetes Toluol verwendet. Als Laufmittel kamen für diesen Vorgang ca. 500 ml getrocknetes Toluol zum Einsatz. Danach wurde das erhaltene Filtrat bis zur Trockene eingeengt.
Chlorbestimmung: < 20 ppm

Gesamte Ausbeute 35 %. Hiervon Isomer A: 54,3 Massen-%, Isomer B. 45,7 Massen-%.

### Gemisch aus (2A) und (2B) (Vergleichsgemisch):

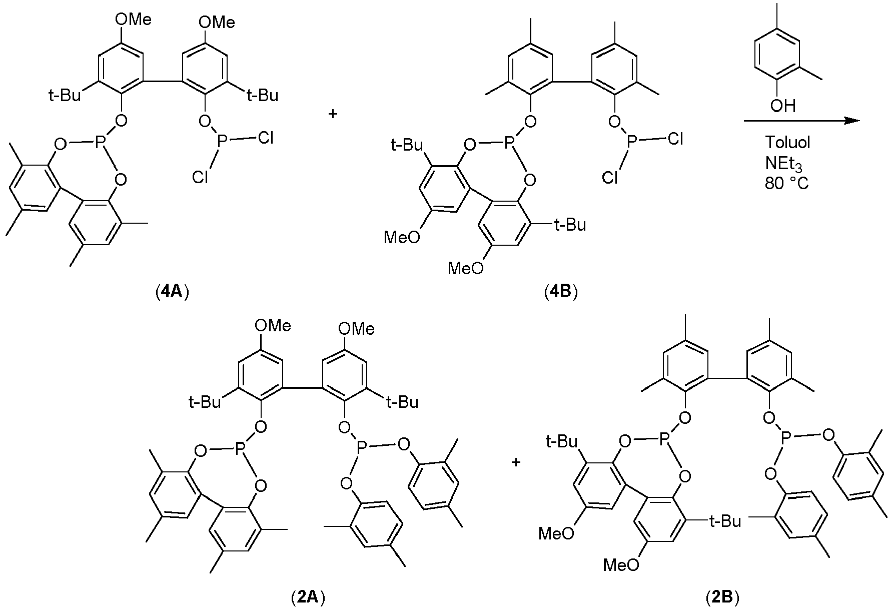

Die Synthese und Aufreinigung erfolgt analog zu (**1A**) und (**1B**), nur dass entsprechend 2.45 g (0,02 mol) 2.4-Dimethylphenol abgewogen wurde.
Gesamte Ausbeute 26 %. Hiervon Isomer A: 59,3 Massen-%, Isomer B. 40,7 Massen-%.

### Katalyseversuche

Die Hydroformylierung wurde in einem mit Druckkonstanthaltung, Gasflussmessung, und Begasungsrührer ausgestatteten 16 ml-Autoklaven der HEL group, Hertfordshire, Großbritannien, durchgeführt. Das als Substrat eingesetzte n-Octen (Oxeno GmbH, Octenisomerengemisch aus 1-Octen: 3 %; cis+trans-2-Octen: 49 %; cis+trans-3-Octen: 29 %; cis+trans-4-Octen: 16 %; gerüstisomere Octene: 3 %) wurde mehrere Stunden über Natrium am Rückfluss erhitzt und unter Argon destilliert.

Für die Versuche wurden die Reaktionslösungen vorab unter Argonatmosphäre vorbereitet. Hierfür wurden 0,0021 g Rh(acac)(CO)₂ und die entsprechende Menge an Phosphitverbindung eingewogen und mit 8,0 ml Toluol aufgefüllt. Die jeweils eingebrachte Masse an Toluol wurde für die GC-Analyse bestimmt. Anschließend wurden 1,80 g n-Octen (16 mmol) hinzugegeben. Die vorbereiteten Lösungen wurden anschließend in den Autoklaven eingefüllt und dieser dreimal mit Argon und dreimal mit Synthesegas (Linde; H₂ (99,999 %) : CO (99,997%) = 1:1) gespült. Dann wurde der Autoklav bei einem Gesamtdruck von 10 bar unter Rühren (900 U/min) auf die angestrebte Temperatur aufgeheizt. Nach Erreichen der Reaktionstemperatur wurde der Synthesegasdruck auf 20 bar erhöht und die Reaktion bei konstantem Druck für 4 h geführt. Der Autoklav wurde nach Ablauf der Reaktionszeit auf Raumtemperatur abgekühlt, unter Rühren entspannt und mit Argon gespült. Jeweils 0,5 ml der Reaktionsmischungen wurden nach Beenden der Reaktion entnommen, mit 4 ml Pentan verdünnt und gaschromatographisch analysiert: HP 5890 Series II plus, PONA, 50 m x 0,2 mm x 0,5 µm. Die quantitative Bestimmung von Restolefin und Aldehyd erfolgte gegen das Lösungsmittel Toluol als interner Standard.

### Ergebnisse der Katalyseversuche

[Rh]: 120 ppm, L:Rh = 1:2, p: 20 bar, T: 120 °C; t: 4 h

**Tabelle: Hydroformylierung der n-Octene**

| Liganden-Gemische | n/iso-Selektivität in % | Ausbeute |
|---|---|---|
| **1A + 1B*** | 78 | 21 % |
| **2A + 2B** | 75 | 17 % |

| | | |
|---|---|---|
| * erfindungsgemäßes Gemisch | | |

### Definition der Selektivität:

Bei der Hydroformylierung gibt es die n/iso-Selektivität: das Verhältnis von linearem Aldehyd (= n) zu verzweigtem Aldehyd (= iso). Hierbei bedeutet die Selektivität bezüglich des n-Aldehyden, dass diese Menge an linearem Produkt gebildet wurde. Die restlichen Prozente entsprechen dann dem verzweigten Isomer. Bei einer Regioselektivität von 50% entstehen also n-Aldehyd und iso-Aldehyd zu gleichen Teilen.

Mit dem erfindungsgemäßen Gemisch (**1A**) und (**1B**) konnte eine gegenüber dem Vergleichsgemisch aus (**2A**) und (**2B**) gesteigerte Selektivität und Ausbeute erzielt werden.

Die durchgeführten Versuche belegen, dass die gestellte Aufgabe durch ein erfindungsgemäßes Gemisch gelöst wird.

## Patentansprüche

1. Gemisch umfassend die Verbindungen (**1A**) und (**1B**):

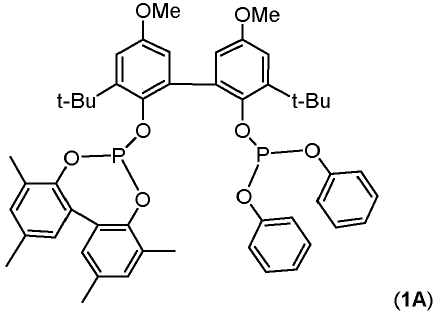

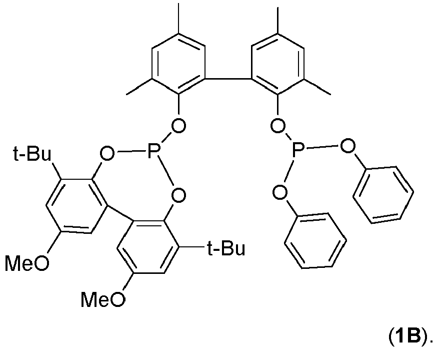

2. Gemisch nach Anspruch 1,
wobei der Gehalt an Verbindung (**1A**) in einem Bereich von 99,5 bis 0,5 Massen-%, der Gehalt an Verbindung (**1B**) in einem Bereich von 0,5 bis 99,5 Massen-% liegt.

3. Gemisch nach einem der Ansprüche 1 oder 2,
wobei der Gehalt an Verbindung (**1A**) in einem Bereich von 75 bis 25 Massen-%, der Gehalt an Verbindung (**1B**) in einem Bereich von 25 bis 75 Massen-% liegt.

4. Gemisch nach einem der Ansprüche 1 bis 3,
wobei der Gehalt an Verbindung (**1A**) bezogen auf Massen-% größer ist, als der Gehalt an Verbindung (**1B**).

5. Verwendung eines Gemisches nach einem der Ansprüche 1 bis 4,
zur Katalyse einer Hydroformylierungsreaktion.

6. Verfahren umfassend die Verfahrensschritte:
a) Vorlegen eines Olefins,
b) Zugabe eines Gemisches nach einem der Ansprüche 1 bis 4 und einer Substanz, welche ein Metall ausgewählt aus: Rh, Ru, Co, Ir aufweist,
c) Zuführen von H₂ und CO,
d) Erwärmen des Reaktionsgemisches aus a) bis c), wobei das Olefin zu einem Aldehyd umgesetzt wird.

## Claims

1. Mixture comprising the compounds (**1A**) and (**1B**):

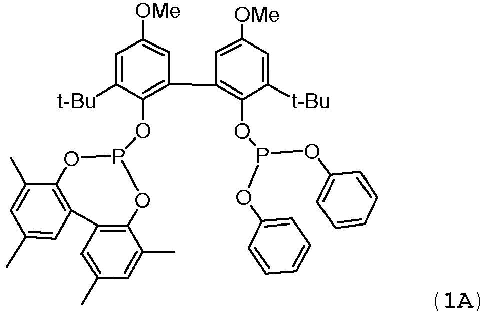

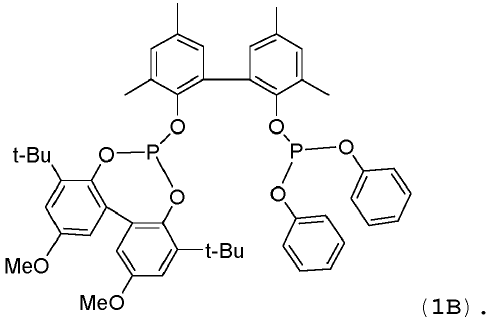

2. Mixture according to Claim 1,
wherein the content of compound (**1A**) is in a range from 99.5% to 0.5% by mass and the content of compound (**1B**) is in a range from 0.5% to 99.5% by mass.

3. Mixture according to either of Claims 1 or 2,
wherein the content of compound (**1A**) is in a range from 75% to 25% by mass and the content of compound (**1B**) is in a range from 25% to 75% by mass.

4. Mixture according to any of Claims 1 to 3,
wherein the content of compound (**1A**) in terms of % by mass is greater than the content of compound (**1B**).

5. Use of a mixture according to any of Claims 1 to 4 for catalysis of a hydroformylation reaction.

6. Process comprising the process steps of:
a) initially charging an olefin,
b) adding a mixture according to any of Claims 1 to 4 and a substance comprising a metal selected from: Rh, Ru, Co, Ir,
c) supplying H₂ and CO,
d) heating the reaction mixture from a) to c), to convert the olefin into an aldehyde.

## Revendications

1. Mélange comprenant les composés (**1A**) et (**1B**) :

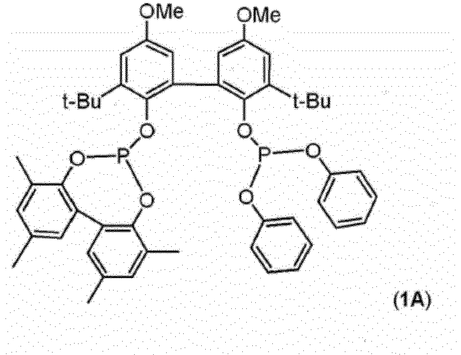

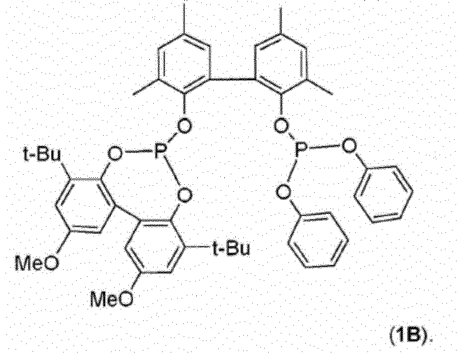

2. Mélange selon la revendication 1, dont la teneur en le composé (**1A**) est comprise dans la plage de 99,5 à 0,5 % en masse, la teneur en le composé (**1B**) est comprise dans la plage de 0,5 à 99,5 % en masse.

3. Mélange selon l'une des revendications 1 ou 2, dont la teneur en le composé (**1A**) est comprise dans la plage de 75 à 25 % en masse, la teneur en le composé (**1B**) est comprise dans la plage de 25 à 75 % en masse.

4. Mélange selon l'une des revendications 1 à 3, dont la teneur en le composé (**1A**), exprimée en % en masse, est supérieure à la teneur en le composé (**1B**).

5. Utilisation d'un mélange selon l'une des revendications 1 à 4 pour la catalyse d'une réaction d'hydroformylation.

6. Procédé comprenant les étapes suivantes :
a) mise en place d'une oléfine,
b) addition d'un mélange selon l'une des revendications 1 à 4 et d'une substance comprenant un métal choisi parmi Rh, Ru, Co, Ir,
c) amenée de H₂ et de CO,
d) chauffage du mélange réactionnel de a) à c), l'oléfine étant convertie en un aldéhyde.
